# EUROPEAN PATENT APPLICATION

(11) **EP 2 253 283 A1**
(43) Date of publication of application: **24.11.2010**
(21) Application number: 10250944.5
(22) Date of filing: 18.05.2010
(51) Int. Cl.: A61B 17/34

(54) **Flexible access assembly with reinforced lumen**

(30) Priority: 19.05.2009 US 179446 P; 20.04.2010 US 763241
(71) Applicant: Tyco Healthcare Group LP, North Haven, CT 06473 (US)
(72) Inventor: Stellon, Gene A., Burlington, CT 06013 (US); Berry, Danny, Hamden, CT 06514 (US); Richard, Paul D., Shelton, CT 06484 (US); Necio, Caren, Hamden, CT 06517 (US)
(74) Representative: Soames, Candida Jane

(57) **Abstract**

An access assembly (10,110) for insertion through a single incision is provided. The access assembly includes a foam body (12,112) having a proximal end and a distal end and a plurality of lumen (16,18,20,116,118,120) extending through the foam body, each of the lumen including a sleeve (16a,18a,20a,116a,118a,120a) extending the length of the body.

## Description

### CROSS REFERENCE TO RELATED APPLICATION

The present application claims the benefit of and priority to U.S. Provisional Application Serial No. 61/179,446 filed on May 19, 2009, the entire contents of which are incorporated herein by reference.

### BACKGROUND

### Technical field

The present disclosure relates to a flexible access assembly for use in single incision surgical procedures. More particularly, the present disclosure relates to a flexible access assembly having multiple instrument lumen.

### Background Of Related Art

Methods and apparatus for performing closed surgical procedures are known. Such procedures greatly reduce postoperative recovery time and minimize scarring to the patient. These procedures typically involve inserting one or more access assemblies through the abdominal wall of the patient and insufflating the abdominal cavity. A laparoscope or other viewing instrument is inserted through one of the access assemblies, or directly through the abdominal wall, to provide the clinician with an image of the abdominal cavity. The clinician is then able to perform the procedure within the abdominal cavity by manipulating instruments that have been extended through the access assemblies.

The number and type of instruments that a clinician may use to complete a closed procedure is limited by the number, size and configuration of the access assemblies that have been inserted into the abdominal cavity. Because traditional access assemblies are configured to provide access for only a single instrument, the simultaneous use of any additional instruments requires a corresponding access assembly. For each additional access assembly necessary to complete the procedure, an additional incision must be created. Each additional incision increases the length of the procedure, and may prolong post-operative recovery time.

Therefore, it is desirable to provide an access assembly for insertion through a single incision in the body of a patient which provides multiple lumen for receipt of one or more surgical instruments.

### SUMMARY

Accordingly, provided is an access assembly for insertion through a single incision. The access assembly includes a body, e.g., a foam body, having a proximal end and a distal end and a plurality of lumen extending through the foam body, each of the lumen including a sleeve extending at least a portion of the length of the body.

The foam body may include a central portion and an upper rim at a proximal end of the central portion. The body may have a lower rim at a distal end of the central portion and an upper rim may have a diameter greater than a diameter of the central portion. The access assembly may include three lumen. The sleeves may be integrally formed with the body, or instead, the sleeves may be securely affixed with the body. The sleeves may be formed from one polymer and plastic. The sleeves may define a circular cross-section. The sleeves may include a braided material. The access assembly may further include one or more cannula assemblies inserted through the plurality of lumen. The body may include a Parylene coating. Various other coatings, e.g., hydrophilic, hydrophobic, bio-agents, anti-infection, analgesic, may also be employed.

Further provided is a method of accessing a body cavity. The method includes the steps of creating an incision through the abdominal wall, providing an access assembly having a flexible body and a plurality of lumen extending through the flexible body, each of the lumen including a sleeve extending the length of the body, compressing the flexible body such that it may be inserted through the incision, inserting the compressed body through the incision, releasing the compressed body to permit the body to return towards an original shape, and receiving a cannula assembly through one or more of the plurality of lumen.

The method may further include the step of receiving one or more instruments through the one or more cannula assemblies and removing the one or more cannula assemblies. The method may further include the step of receiving a valve assembly through one of the plurality of lumen. The body may include a Parylene coating. Various other coatings, e.g., hydrophilic, hydrophobic, bio-agents, anti-infection, analgesic, may also be employed.

The invention may be described by reference to the following numbered paragraphs:-

An access assembly, having a foam body and a plurality of lumen extending through the body, each of the lumen including a sleeve extending the length of the body; for use in a method of accessing a body cavity, which method comprises the steps of:
creating an incision through the abdominal wall;
compressing the body such that it may be inserted through the incision;
inserting the compressed body through the incision;
releasing the compressed body to permit the body to return towards an original shape; and
receiving a cannula assembly through one or more of the plurality of lumen.

The access assembly according to paragraph 11, wherein the method further includes the step of receiving one or more instruments through the one or more cannula assemblies and/or the access assembly according to paragraph 11, wherein the method further includes the step of removing the one or more cannula assemblies and/or the access assembly of paragraph 11, wherein the method further includes the step of receiving a valve assembly through one of the plurality of lumen.

The access assembly of any of paragraphs 11 to 12, wherein the body includes a Parylene coating.

### DESCRIPTION OF THE DRAWINGS

Embodiments of a flexible access assembly are disclosed herein with reference to the drawings, wherein:

FIG. 1 is a perspective view of an embodiment of an access assembly according to the present disclosure;

FIG. 2 is a top view of the access assembly of FIG. 1;

FIG. 3 is a cross-sectional side view of the access assembly of FIGS. 1 and 2 taken along line 3-3 of FIG. 2;

FIG. 4 is a perspective view of a tissue section having an incision therethrough with an underlying body organ shown in phantom;

FIG. 5 is a perspective view of the access assembly of FIG. 1 prepared for insertion through the incision in the tissue;

FIG. 6 is a perspective view of the flexible access assembly of FIG. 1 positioned through the incision in the tissue;

FIG. 7 is a side view, partially shown in cross-section, of the access assembly of FIG. 1, including a stopcock valve and a pair of cannula assemblies received therethrough;

FIG. 8 is a perspective view of an alternative embodiment of an access assembly according to the present disclosure;

FIG. 9 is a top view of the access assembly of FIG. 10; and

FIG. 10 is a cross-sectional side view of the access assembly of FIGS. 9 and 10 taken along line 10-10 of FIG. 9.

### DETAILED DESCRIPTION

Embodiments of the presently disclosed access assembly will now be described in detail with reference to the drawings wherein like numerals designate identical or corresponding elements in each of the several views. As is common in the art, the term "proximal" refers to that part or component closer to the user or operator, i.e. surgeon or physician, while the term "distal" refers to that part or component further away from the user.

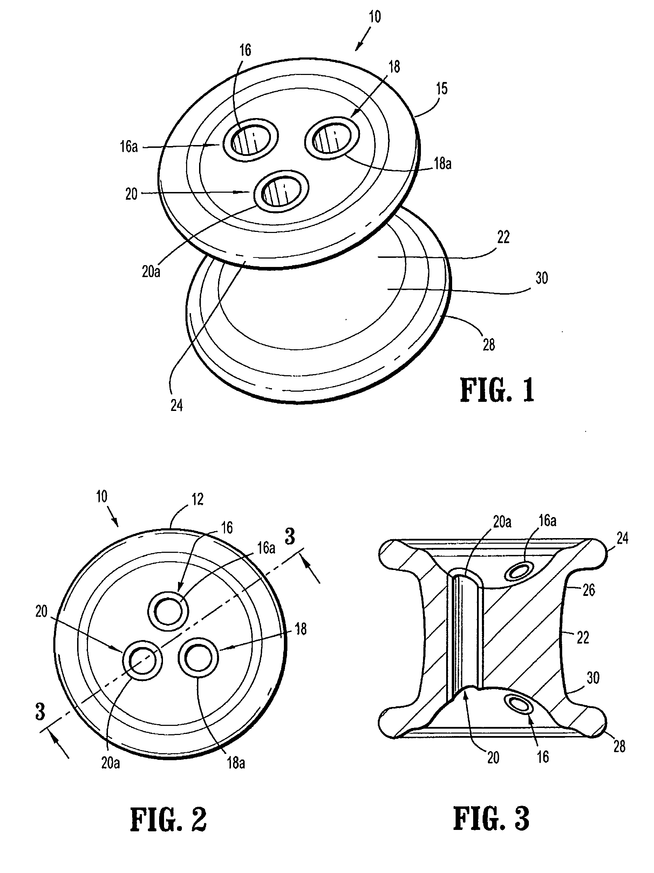

Referring to FIGS. 1-3, there is disclosed an access assembly 10 for use in single incision surgery. Access assembly 10 is flexible or compressible to allow it to be inserted through a single incision in the body of a patient such that after insertion it will expand and seal within the incision. Additionally, the flexible nature of access assembly 10 allows surgical instruments inserted therethrough to be manipulated about their axes and thus allow a higher degree of movement of the surgical instruments to orient them relative to the tissue being operated upon.

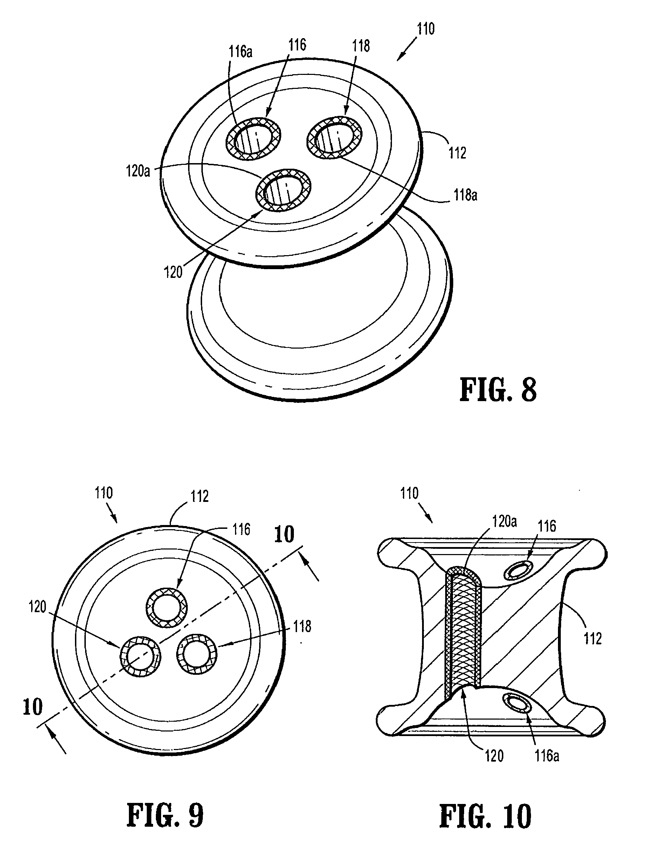

Still referring to FIGS. 1-3, access assembly 10 includes a flexible body or housing 12 defining a plurality of lumen 16, 18, 20. Body 12 may be formed of various materials such as, for example, silicone, thermoplastic elastomers (TPE), rubber, foam, gel, etc. In this manner, body 12 of access assembly 10 may be compressed or squeezed and inserted through an incision in the body of a patient. In one embodiment, body 12 includes TPE material that is infused with an inert gas, e.g. CO₂ or Nitrogen, to form a foam structure. Body 12 may be coated with a lubricant, e.g. Parylene N or C, in order to create a lubricious surface finish on all external surface. Various other coatings, e.g., hydrophilic, hydrophobic, bio-agents, anti-infection, analgesic, may also be employed. In this manner, the coating facilitates insertion of body 12 into an incision and insertion of cannula assemblies (FIG. 8) therethrough.

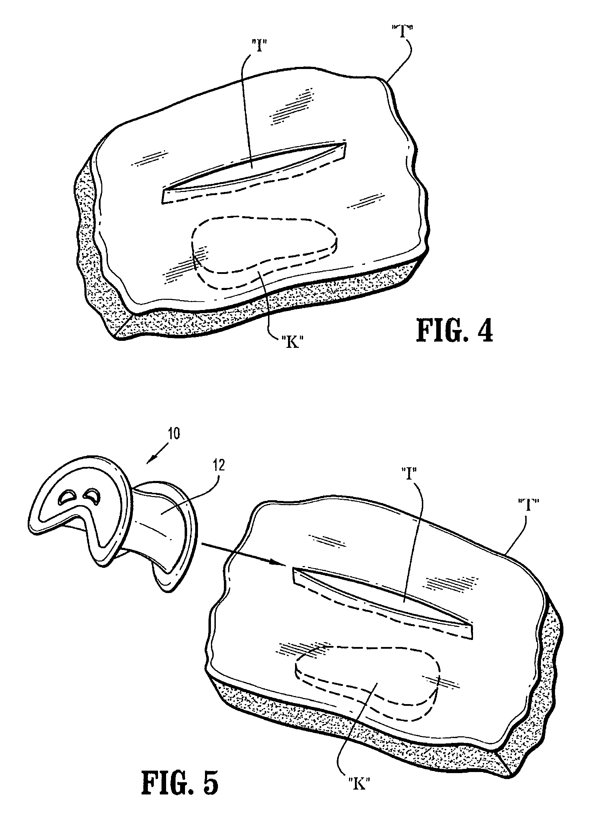

With reference still to FIGS. 1-3, body 12 defines a substantially hourglass shape when viewed from the side, including a central portion 22 having an upper rim 24 located at a proximal end 26 of central portion 22 and a lower rim 28 located at a distal end 30 of central portion 22. Upper rim 24 and lower rim 28 aid in preventing movement of access assembly 10 longitudinally through the incision "I" (FIG. 4) in the patient.

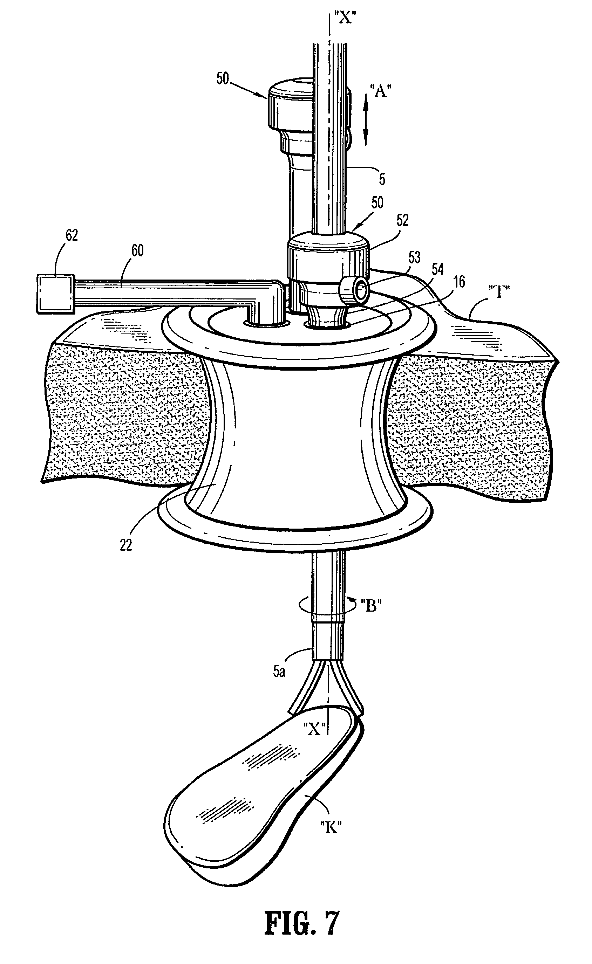

Lumen 16, 18, 20 extend through body 12 and define longitudinal axes configured to receive a cannula assembly 50 (FIG. 7), a valve assembly 60 and/or other insufflation apparatus. As shown, lumen 16, 18, 20 include sleeves 16a, 18a, 20a, respectively, extending the length of body 12. Sleeves 16a, 18a, 20a may be integrally formed with body 12, or instead may be securely affixed to body 12 using adhesive, ultrasonic welding or other suitable means. Sleeves 16a, 18a, 20a are formed of a plastic, polymer or other suitable material and are configured to prevent tearing of body 12 as a cannula assembly or other apparatus is inserted therethrough. Sleeves 16a, 18a, 20a are typically formed of a harder or less flexible material than body 12 to resist stretching. Sleeves 16a, 18a, 20a may also be coated with a lubricant to assist in insertion of cannula assemblies 50 and/or valve assembly 60.

Referring now to FIGS. 4-7, the use of access assembly 10 in a single incision surgical procedure will now be described. Although access assembly 10 will be described as relates to relates to a procedure for excising and removing a body organ, the aspects of the present disclosure may be modified for use in any closed procedure and should not be read as limited to the procedure herein described.

Referring initially to FIG. 4, a single incision "I" is formed through a body tissue "T" and above a body organ, such as, for example, kidney "K". Turning now to FIG. 5, once incision "I" has been formed through body tissue "T", body 12 of access assembly 10 is squeezed or compressed to reduce body 12 to a relatively smaller diameter for insertion through incision "I". As noted hereinabove, body 12 is formed of a flexible material which allows access assembly 10 to be compressed. It should be recognized that the body 12 may be compressed into any suitable configuration prior to being inserted into an incision, not merely the configuration shown in FIG. 5. For example, in an embodiment, prior to insertion the body 12 is clamped at its distal end while the proximal end of the housing 12 remains essentially uncompressed, and the clamped distal end is inserted into the incision.

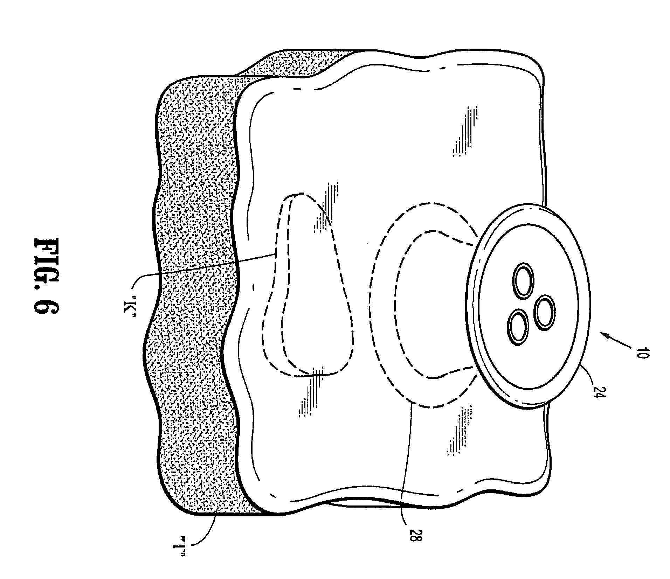

Referring to FIG. 6, once flexible access assembly 10 has been inserted through incision "I", pressure on body 12 is released, allowing body 12 to return towards its initial uncompressed state within incision "I". Typically, the incision "I" is formed having a size that is smaller than the diameter of the initial uncompressed state of the housing 12. In this manner, when in place within the incision "I", the housing 12 contacts and presses against the inner surface of the incision "I", thereby retracting the opening and sealing with the incision "I". Since incisions are often slit-shaped when formed, the portion of the housing 12 that is located within the incision may be somewhat oval-shaped (when viewed from above). As noted hereinabove, body 12 includes upper rim 24 and lower rim 28 to prevent migration of access assembly 10 through incision "I" in body tissue "T".

Turning to FIG. 7, once access assembly 10 has been positioned above kidney "K", cannula assemblies 50 and/or valve assembly 60 may be inserted through seal lumen 16, 18, 20 to operate on kidney "K". Cannula assembly 50 includes a housing 52 configured to sealingly receive an instrument 5 and an elongated cannula 52 configured to extend through one of lumen 16, 18, 20. Housing 52 may include an insufflation port 53. Although shown including cannula assemby 50, any cannula assembly capable of being received through lumen 16, 18, 20 may be used with access assembly 10. Valve assembly 60 is configured to be received through one of lumen 16, 18, 20. Valve assembly 60 may include a stopcock or other type of valve 62 for selectively providing insufflation gas through access assembly 10. Although shown including valve assembly 60, any valve assembly capable of sealed reception within lumen 16, 18, 20 may be used with access assembly 10.

Still referring to FIG. 7, once the body cavity has been properly insufflated, either through valve assembly 60 or insufflation port 53 of cannula assembly 50, kidney "K" may be operated upon to excise it from the surrounding tissue. One or more surgical instruments, such as, for example, tissue graspers or surgical staplers, are inserted through and manipulated within cannula assemblies 50 to complete the procedure. As shown, instrument 5 may be inserted and retracted, in the direction of arrows "A", through any of seal lumen 16, 18, 20 that have received a cannula assembly 50 therethrough. Due to the flexible nature of access assembly 10, cannula assembly 50 may be flexed relative thereto. In this manner, once instrument 5 is inserted through cannula assembly 50, a proximal end 5a of instrument 5 may be manipulated in any direction, as indicated by arrows "B". Thus, access assembly 10 permits a surgeon to manipulate or orient instrument 5 at various locations relative to the tissue being operated upon. Cannula assemblies 50 may also be flexed relative to each other. In this manner, a first instrument inserted through a first cannula assembly may be manipulated relative to a second instrument inserted through a second cannula assembly.

Upon completion of the procedure, cannula assemblies 50 and valve assembly 60 are removed from respective lumen 16, 18, 20. Access assembly 10 is then compressed or squeezed such that it may be removed from incision "I". Incision "I" is then closed in a conventional manner.

Turning now to FIGS. 4-6, an alternative embodiment of an access assembly according to the present disclosure is shown generally as access assembly 110. Access assembly 110 is substantially similar to access assembly 10 described hereinabove, and will only be described as relates to the differences therebetween. Access assembly 110 includes a body 112 defining a plurality of lumen 116, 118, 120. Each of lumen 116, 118, 120 includes a sleeve 116a, 118a, 120a, respectively. Each of sleeves 116a, 118a, 120a is formed of a braided mesh. As with sleeves 16a, 18a, 20a, described hereinabove, sleeves 116a, 118a, 120a are configured to prevent tearing of body 112 as cannula assembly and other apparatus are inserted therethrough.

It will be understood that various modifications may be made to the embodiments disclosed herein. For example, as noted hereinabove, the disclosed flexible access assembly may be provided with multiple lumen in excess of the disclosed three lumen. Additionally, the diameters or configuration of the disclosed lumen need not be identical but may be varied depending upon the contemplated surgical instruments to be utilized therethrough. Therefore, the above description should not be construed as limiting, but merely as exemplifications of particular embodiments. Those skilled in the art will envision other modifications within the scope and spirit of the claims appended hereto.

## Claims

1. An access assembly for insertion through tissue, the access assembly comprising:
a foam body having a proximal end and a distal end; and
a plurality of lumen extending through the flexible body, each of the lumen including a sleeve extending at least a portion of the length of the body.

2. The access assembly as recited in claim 1, wherein the body has a central portion and an upper rim at a proximal end of the central portion.

3. The access assembly as recited in claim 2, wherein the body has a lower rim at a distal end of the central portion.

4. The access assembly as recited in claim 2, wherein the upper rim has a diameter greater than a diameter of the central portion.

5. The access assembly as recited in any preceding claim, including three lumen.

6. The access assembly as recited in any preceding claim, wherein the sleeves are integrally formed with the body.

7. The access assembly as recited in any of claims 1 to 5, wherein the sleeves are securely affixed with the body.

8. The access assembly as recited in any preceding claim, wherein the sleeves define a circular cross-section.

9. The access assembly as recited in any preceding claim, further including one or more cannula assemblies inserted through the plurality of lumen.

10. The access assembly as recited in any preceding claim, wherein the sleeves are formed from one of polymer and plastic.

11. The access assembly as recited in any preceding claim, wherein the sleeves include a braided material.

12. The access assembly as recited in any preceding claim, wherein the body includes a coating that is at least one of parylene, hydrophilic, hydrophobic, bio-agents, anti-infection and analgesic.

13. An access assembly according to any preceding claim, having a foam body and a plurality of lumen extending through the body, each of the lumen including a sleeve extending the length of the body; for use in a method of accessing a body cavity, which method comprises the steps of:
creating an incision through the abdominal wall;
compressing the body such that it may be inserted through the incision;
inserting the compressed body through the incision;
releasing the compressed body to permit the body to return towards an original shape;
and
receiving a cannula assembly through one or more of the plurality of lumen.

14. The access assembly according to claim 13, wherein the method further includes the step of receiving one or more instruments through the one or more cannula assemblies and/or the access assembly according to claim 13, wherein the method further includes the step of removing the one or more cannula assemblies and/or the access assembly of claim 13, wherein the method further includes the step of receiving a valve assembly through one of the plurality of lumen.

15. The access assembly of any of claims 13 to 14, wherein the body includes a Parylene coating.
